# FASCICULE DE BREVET EUROPEEN

(11) **EP 0 760 142 B1**
(45) Date de publication et mention de la délivrance du brevet: **04.08.1999**
(21) Numéro de dépôt: 95920936.2
(22) Date de dépôt: 11.05.1995
(51) Int. Cl.: G06F 19/00, G06F 159/00

(54) **DISPOSITIF DE TRANSCRIPTION ET D'ENREGISTREMENT DE LA POSOLOGIE D'UN MEDICAMENT PRESCRIT**
EINRICHTUNG ZUM VERSCHREIBEN UND AUFZEICHNEN VON DOSIERUNGSANLEITUNGEN FÜR ARZNEIMITTEL
DEVICE FOR TRANSCRIBING AND RECORDING THE DOSAGE INSTRUCTIONS OF A PRESCRIPTION DRUG

(30) Priorité: 11.05.1994 FR 9405812
(43) Date de publication de la demande: 05.03.1997
(73) Titulaire: MANUFACTURE D'APPAREILLAGE ELECTRIQUE DE CAHORS, F-46003 Cahors Cédex (FR); Vie, Philippe, 31000 Toulouse (FR); Peyre, Alain, 31000 Toulouse (FR)
(72) Inventeur: VIE, Philippe, F-31000 Toulouse (FR); PEYRE, Alain, F-31000 Toulouse (FR)
(74) Mandataire: Derambure, Christian
(86) Numéro de dépôt international: FR9500621
(87) Numéro de publication internationale: WO9531790

(56) Documents cités:
- EP-A- 0 212 759
- EP-A- 0 283 752
- WO-A-91/19964
- DE-U- 9 314 370
- PATENT ABSTRACTS OF JAPAN vol. 11 no. 391 (P-649) ,22 Décembre 1987 & JP,A,62 156771 (TOKYO SHOKAI K.K.) 11 Juillet 1987,
- IBM TECHNICAL DISCLOSURE BULLETIN., vol. 18, no. 6, Novembre 1975 NEW YORK US, page 19671972 DICKERSON ET AL. 'Hospital Comunications System'

## Description

L'invention concerne un dispositif de transcription et d'enregistrement de la posologie d'un médicament prescrit.

On connaît le document "IBM T.D.B.v.18, n° 6, p. 1967-1972 ;Nov 1985: Dickerson et al.: Hospital Communications System" qui décrit un système comprenant des moyens de transcription et d'enregistrement de la forme galénique d'un médicament, de sa fréquence, du moment et de la qualité de prise, de la durée du traitement, du type de malade traité, des moyens de traitement de l'information et de la posologie, et des moyens d'impression.

Les problèmes pratiques générés par la consommation de médicaments ont presque toujours pour origine l'interprétation de l'ordonnance prescrite par le médecin. Le grand public est confronté de façon générale, à des problèmes de deux ordres :
- des problèmes qui, s'ils ne sont pas résolus, affectent directement la sécurité du malade.

C'est le cas des problèmes liés à une mauvaise lecture de l'ordonnance entraînant des erreurs quantitatives et qualitatives au niveau de la prise du médicament; des erreurs dans le moment de la prise et des inversions de médicaments.

C'est le cas également des problèmes liés à une erreur d'attribution du médicament, notamment dans le cas où l'on destine à un nourrisson ou à un enfant un médicament strictement réservé à un adulte. Ce type d'erreur peut intervenir soit immédiatement après la prescription du traitement, soit longtemps après, les médicaments entamés étant souvent conservés dans l'armoire à pharmacie des particuliers.

Ces problèmes sont d'autant plus présents que de plus en plus de personnes se soignent elles-mêmes sans avis médical préalable.
- des problèmes pratiques liés à la mauvaise lisibilité de l'ordonnance et à la nécessité de se reporter constamment à l'ordonnance lors de chaque prise de médicaments.

Le public reste très sensible à ces deux aspects problématiques de la consommation médicamenteuse.

Le pharmacien d'officine tente aujourd'hui de pallier ces problèmes, par l'écriture directe sur le conditionnement du médicament ou, lorsque le temps le permet, l'écriture sur une étiquette, ensuite collée sur ce conditionnement.

En tout cas, les problèmes de lecture ne sont pas totalement résolus, le pharmacien perd son temps sans pouvoir raisonnablement mentionner la destination de chaque médicament en fonction de chaque membre d'une famille.

L'objet de l'invention est un dispositif selon les revendications, qui permet de pallier l'ensemble de ces inconvénients.

Le préambule de la revendication 1, correspond à la teneur du document évoqué plus haut. L'invention propose un dispositif de transcription et d'enregistrement de la posologie d'un médicament prescrit, qui, en combinaison, permette au pharmacien d'officine de répondre de façon sûre et rapide aux problèmes de sécurité et du confort de son client. Ces deux problèmes sont majeurs et constituent le fondement de la mission des pharmaciens au sein du service public.

Ces problèmes sont résolus selon l'invention par un dispositif tel que défini dans la revendication 1 et un procédé tel que défini dans la revendication 8.

Ce dispositif de transcription, d'enregistrement et de restitution de la posologie d'un médicament prescrit, comprend un module électronique commandant notamment des moyens de transcription et d'enregistrement de la forme galénique du médicament, des moyens de transcription et d'enregistrement de la fréquence de prise de médicament, des moyens de transcription et d'enregistrement de la quantité de prise de médicament, des moyens de transcription et d'enregistrement de la durée de traitement par le médicament, des moyens de traitement des éléments d'information et/ou de la posologie transcrits et enregistrés.

Ce dispositif comporte en outre des moyens de transcription et d'enregistrement du moment de la prise du médicament, des moyens de transcription et d'enregistrement du type de malade traité, et des moyens d'impression.

Un tel dispositif est connu comme partie intégrante d'un système onéreux et complexe.

Le dispositif permet l'impression d'éléments d'information et la posologie transcrits et enregistrés sur une étiquette adaptée à être fixée sur la boite contenant ledit médicament.

On obtient ainsi un dispositif très simple, qui peut ainsi être économique et fiable, et qui apporte une solution satisfaisante aux problèmes précités des pharmaciens d'officine et de leurs clients auxquels ont été prescrits un ou plusieurs médicaments.

Ce dispositif imprime une étiquette qui est adaptée à être fixée par le pharmacien sur la boite contenant ledit médicament, et qui comporte uniquement les informations dont a besoin le patient pour prendre ce médicament selon les prescriptions du médecin.

Suivant une version simple de l'invention, les moyens de transcription et d'enregistrement et les moyens de traitement sont matérialisés par des touches correspondant chacune respectivement à un élément d'information composant la posologie du médicament. Ces touches peuvent comporter sur leur face externe respective un pictogramme illustrant l'élément d'information à transcrire et enregistrer, et présenter plusieurs branches.

Les autres caractéristiques et avantages de l'invention seront bien compris grâce à la description qui suit en référence aux dessins annexés dans lesquels :
- la figure 1 est une représentation schématique et fonctionnelle d'un dispositif de transcription et d'enregistrement de la posologie d'un médicament prescrit conforme à la présente invention.
- la figure 2 est une vue de dessus d'un mode de réalisation particulier d'un dispositif de transcription et d'enregistrement de la posologie d'un médicament prescrit conforme à la présente invention.

L'invention concerne donc un dispositif de transcription et d'enregistrement de la posologie d'un médicament prescrit. Il est donc destiné aux pharmaciens d'officine et, à cet effet, doit remplir un cahier des charges sévère tant sur le plan de sa taille, sa maniabilité, sa fiabilité, que sur le plan de sa rapidité.

Le dispositif selon l'invention comprend tout d'abord un module électronique 1 ayant pour fonction notamment le traitement des signaux issus des moyens de transcription et d'enregistrement des éléments d'information composant la posologie du médicament, la mise en oeuvre d'un programme en mémoire morte par le microprocesseur permettant la gestion de l'ensemble du dispositif et l'interface imprimante.

Ce module électronique 1 commande notamment des moyens 2 de transcription et d'enregistrement de la forme galénique du médicament, des moyens 3 de transcription et d'enregistrement de la fréquence de prise du médicament, des moyens 4 de transcription et d'enregistrement de la quantité de prise de médicament, des moyens 5 de transcription et d'enregistrement du moment de la prise de médicament, et des moyens 6 de transcription et d'enregistrement du type de malade traité.

Le dispositif conforme à l'invention peut comprendre en outre un moyen 7 de transcription et d'enregistrement de la durée du traitement par le médicament ainsi que des moyens de traitement 9 des éléments d'information et/ou de la posologie transcrits et enregistrés.

Selon l'invention, les moyens de transcription et d'enregistrement 1 à 7 et les moyens de traitement 9 sont matérialisés par des touches 8, 8a correspondant à un élément d'information composant la posologie du médicament.

De manière à accélérer la manipulation du dispositif conforme à l'invention, les touches 8, 8a comportent sur leur face externe un pictogramme illustrant l'élément d'information sélectionné.

Ces touches 8 ont toutes la capacité d'enregistrer un élément d'information composant la posologie du médicament à mémoriser par simple pression. Elles sont interdépendantes et étudiées pour émettre un signal différent selon l'ordre d'entrée des autres éléments d'informations nécessaires à la transcription totale de la posologie.

Leur emplacement a été analysé et déterminé afin de respecter la logique du message à transcrire et de permettre une mémorisation rapide de leur emplacement sur un clavier.

L'optimisation fonctionnelle de l'ensemble du clavier permet la restitution d'un message formé de caractères (huit en moyenne) par pression sur une seule touche 8, soit en moyenne quatre à cinq frappes par transcription d'une posologie d'un médicament, ce qui peut être simple, rapide et fiable.

Le programme contenu dans la mémoire morte du module électronique 1, et mis en oeuvre par le microprocesseur de ce module, impose cette logique de transcription sous la forme d'un protocole de transcription imposant la séquence et le mode de transcription des éléments d'information à transcrire.

Ce protocole peut ainsi imposer d'entrer successivement les informations suivantes :
- type de malade ;
- forme galénique ;
- quantité ;
- fréquence de prise ;
- moment de la prise ;
- durée du traitement.

Seules les informations se présentant sous une forme prédéterminée compatible avec la rubrique concernée sont enregistrées par le dispositif.

Chaque touche correspond à un message prédéterminé enregistré dans la mémoire morte et susceptible d'être restitué.

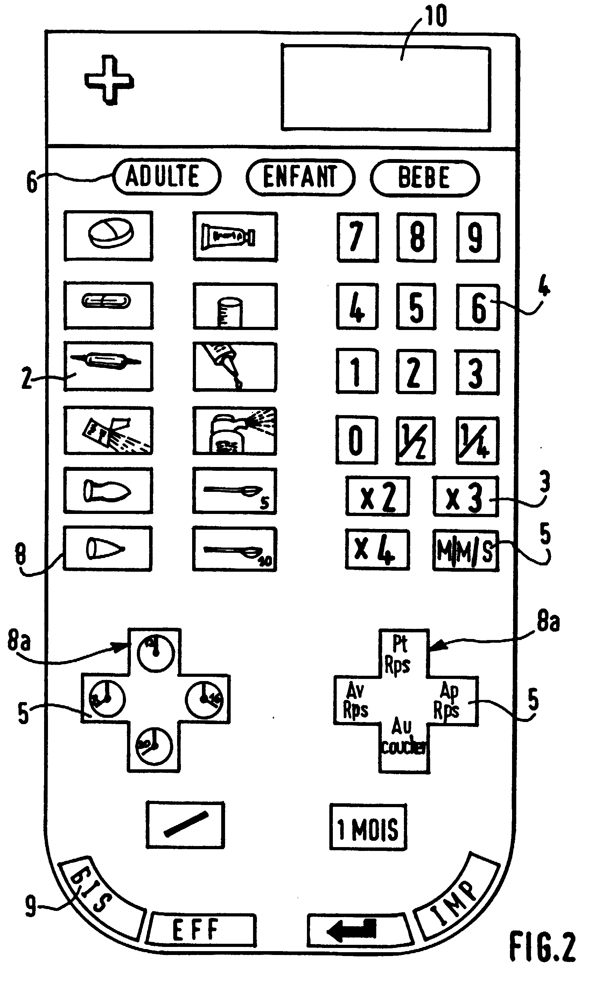

Dans un mode de réalisation préféré, illustré par la figure 2, les touches 8 matérialisant les moyens 2 de transcription et d'enregistrement de la forme galénique du médicament sont au nombre de douze et comportent sur leur face externe un pictogramme représentant un comprimé, une gélule, une ampoule, un -sachet, une dose, une cuillère à café, une cuillère à soupe, un ovule, un suppositoire, des gouttes, une pommade-crème, un pulvérisateur.

Les touches 8 matérialisant les moyens 3 de transcription et d'enregistrement de la fréquence de prise du médicament sont par exemple au nombre de trois et peuvent délivrer des éléments d'information composant la posologie à transcrire venant s'articuler sur les précédents éléments d'information, se différenciant et s'adaptant suivant l'ordre de frappe.

Les touches 8 matérialisant les moyens 4 de transcription et d'enregistrement de la quantité de prise de médicaments sont sous forme d'un clavier numérique classique comportant deux touches supplémentaires : 1/4 et 1/2.

Les touches 8, 8a matérialisant les moyens 5 de transcription et d'enregistrement du moment de la prise de médicament sont au nombre de neuf par exemple et permettent de transcrire les éléments d'informations de la posologie suivante : avant le repas, pendant le repas, après le repas, au coucher ; 8 heures, 12 heures, 16 heures, 20 heures ; matin, midi, soir.

Les touches 8 matérialisant le moyens de transcription et d'enregistrement du type de malade traité 6 sont, par exemple, au nombre de trois et sont destinés à transcrire les éléments d'information suivants : adulte, enfant, nourrisson.

En l'absence de la précision du type de malade traité, le module électronique 1 est programmé de manière à sélectionner l'élément d'information "adulte". Cette disposition permet de limiter au maximum les risques de surdosages de médicament. Par ailleurs, l'information sur le type de malade concerné, une fois sélectionnée pour le premier médicament d'une ordonnance, sera implicitement conservée pour tous les autres médicaments à transcrire.

Les touches 8 matérialisant le moyens de transcription et d'enregistrement de la durée de traitement 7 sont sous la forme d'un clavier numérique classique et d'une touche supplémentaire enregistrant une durée mensuelle du traitement (touche : "1 mois"). Elles sont destinées à transcrire les éléments d'information du type : pendant 1 mois, pendant 10 jours... Une autre touche pourrait concerner le cas d'un traitement de plusieurs mois.

Les moyens de traitement 9 des éléments d'informations et/ou de la posologie transcrits et enregistrés permettent notamment, la répétition, la relecture, le démarrage de l'impression, etc...

Les touches 8 sont par exemple des touches ordinaires envoyant un signal chaque fois qu'elles reçoivent une pression.

Le dispositif peut comporter également des touches 8a à plusieurs branches, notamment cruciformes, dont on peut presser chaque branche indépendamment des autres branches.

On peut ainsi, au titre des moyens 5 de transcription du moment de la prise du médicament, et comme représenté à la figure 2, avoir une première touche cruciforme 8a dont les différentes branches correspondent respectivement aux diverses heures approximatives habituelles de prise : 8 heures, 12 heures, 16 heures, 20 heures, et une seconde touche cruciforme 8a dont les différentes branches permettent de préciser, par exemple, avant le repas, pendant le repas, après le repas, au coucher. La dernière touche correspond à la prescription matin/midi/soir.

Les touches 8, 8a sont ainsi disposées de haut en bas sur le dispositif de la figure 2, dans l'ordre de transcription imposé par le protocole précité.

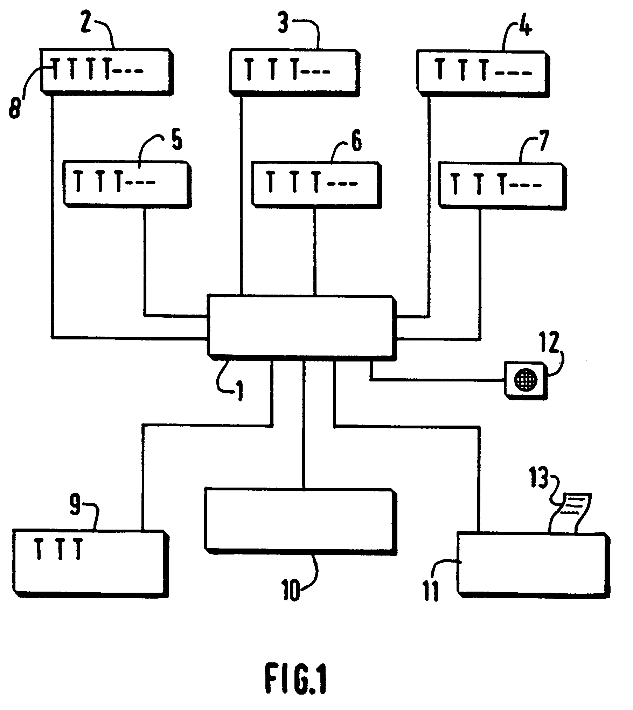

Dans une autre variante de l'invention, les moyens de transcription et d'enregistrement 2 à 7 sont constitués d'un système de reconnaissance vocale. Ce type de système fournit la retranscription intégrale et sans erreur d'un texte oral correspondant à la prescription du médecin. Un microphone utilisé à cet effet et schématisé en 12 à la figure 1 est relié au module électronique 1 du dispositif conforme à l'invention.

Le message posologique transmis oralement, constitué de mots clefs (type de malade, quantité, forme galénique, ...) agencés suivant la même logique que celle établie à l'aide des touches, elle-même conforme à la logique de prescription du médecin, est déchiffré par le module électronique 1 chargé d'un programme adapté.

L'interprétation de ce message par le programme est alors identique à l'interprétation faite suite à un enregistrement réalisé avec l'ensemble des touches 8 pour transcrire totalement une posologie ou une série de posologies.

Là encore, en l'absence de la précision du type de malade traité, le module électronique 1 est programmé de manière à sélectionner l'élément d'information "adulte" dans le but de limiter au maximum les risques de surdosages de médicament.

La variante à reconnaissance vocale du dispositif conforme à l'invention permet donc de pallier complètement l'usage des touches 8, seuls les moyens de traitement 9, de visualisation 10, d'impression 11 des éléments d'information et/ou de la posologie transcrits et enregistrés seront conservés sur le dispositif.

Le dispositif de transcription et d'enregistrement de la posologie d'un médicament prescrit conforme à la présente invention comprend en outre un moyen de visualisation 10 des éléments d'information et/ou de la posologie transcrits et enregistrés. A titre illustratif, ce peut être un écran à cristaux liquides permettant la visualisation des derniers messages traités ou des messages du protocole d'utilisation du dispositif, et la visualisation de la posologie qui doit être imprimée.

Enfin, le dispositif conforme à la présente invention comprend en outre un moyen d'impression il de la posologie transcrite et enregistrée, sous la forme d'une imprimante intégrée ou reliée au dispositif par tous moyens adaptés. Ces moyens d'impression permettent l'impression d'une étiquette 13 destinée à être fixée sur la boîte du médicament correspondant. Le message imprimé comprendra à l'intention du patient qui prend ce médicament ou de la personne qui lui administre ce médicament toutes les informations lui permettant de suivre la prescription du médecin, et uniquement celles-là.

L'impression d'une étiquette contenant uniquement les informations nécessaires au patient ou à une personne ayant en charge le patient est donc une caractéristique essentielle de l'invention. Cette étiquette est ainsi préparée très rapidement par le pharmacien qui reste dans son rôle d'intermédiaire entre le médecin prescripteur et le patient.

La taille du dispositif schématisé à la figure 2 est telle qu'il est susceptible d'être tenu dans une main. Son encombrement peut être comparée à celui d'un combiné téléphonique, la prise manuelle est aisée afin que l'autre main reste disponible pour la manipulation du clavier. Son autonomie électrique en autorise le déplacement sur n'importe quel comptoir ou plan de travail.

Par ailleurs, de nombreuses officines de pharmacie sont maintenant équipées d'un micro-ordinateur qui a pour fonctions principales la gestion des approvisionnements et des stocks de médicaments et autres produits, ainsi que d'autres tâches administratives telles que la tenue des registres obligatoires, par exemple celui des noms et adresses des patients auxquels sont vendus des médicaments dont la vente et la diffusion sont contrôlés.

Un autre mode de réalisation de l'invention consiste évidemment à aménager un tel micro-ordinateur en lui adjoignant les moyens, clavier et/ou logiciel, imprimante pour étiquettes, nécessaires pour que ce micro-ordinateur comporte tous les moyens précités de l'invention et soit capable d'imprimer l'étiquette décrite ci-dessus adaptée à être fixée sur la boite de médicament, conformément au but de l'invention défini dans l'introduction de la présente description.

Ces moyens peuvent être constitués par une disquette contenant un simple logiciel complémentaire adapté à faire apparaître sur l'écran les schémas, pictogrammes et messages représentant d'une part le protocole de transcription des éléments d'information, d'autre part ces éléments d'information, associé à une souris ou à un organe analogue permettant d'enregistrer l'élément d'information adéquat.

Cet organe pourrait être un écran tactile sur lequel apparaîtraient les pictogrammes et messages ou informations représentés à la figure 2 et décrits ci-dessus.

Le système comprenant les moyens matériels et de logiciel correspondants nécessaires pour aménager de cette manière un micro-ordinateur de gestion des approvisionnements et des stocks de médicament d'une officine de pharmacie fait bien entendu partie de la présente invention.

Bien entendu, la présente invention n'est pas limitée aux modes de réalisation décrits ci-dessus, et on peut apporter à ceux-ci de nombreux changements et modifications sans sortir du domaine de l'invention.

On peut ainsi remplacer les touches 8 par d'autres moyens équivalents, et prévoir sur les étiquettes des informations supplémentaires par rapport à celles décrites.

## Revendications

1. Dispositif destiné à être utilisé par un pharmacien, de transcription, d'enregistrement et de restitution de la posologie d'un médicament prescrit à une personne du grand public, comprenant un module électronique (1) commandant notamment des moyens (2) de transcription et d'enregistrement de la forme galénique du médicament, des moyens (3) de transcription et d'enregistrement de la fréquence de prise du médicament, des moyens (4) de transcription et d'enregistrement de la quantité de prise du médicament, des moyens (7) de transcription et d'enregistrement de la durée du traitement par le médicament, des moyens (9) de traitement des éléments d'information et/ou de la posologie transcrits et enregistrés, des moyens (5) de transcription et d'enregistrement du moment de la prise du médicament, des moyens (6) de transcription et d'enregistrement du type de malade traité, et des moyens d'impression (11), caractérisé en ce que les moyens (11) sont reliés aux moyens de traitement (9) pour imprimer uniquement des éléments d'information dont a besoin le patient pour prendre le médicament selon la prescription du médecin, transcrits et enregistrés sur une étiquette adaptée à être fixée sur la boite contenant ce médicament, les moyens de traitement (6) étant programmés de manière à sélectionner par défaut l'élément d'information "adulte" afin de limiter les risques de surdosages de médicament.

2. Dispositif selon la revendication 1, caractérisé en ce que le module électronique (1) comporte une mémoire morte et un microprocesseur qui imposent un protocole de transcription dont les séquences obligent d'entrer successivement les éléments d'information suivants :
• type de malade ;
• forme galénique du médicament;
• quantité;
• fréquence de prise ;
• moment de prise ; et
• durée du traitement ;
et n'enregistre que les éléments d'information qui sont présentés sous une forme prédéterminée compatible avec la rubrique concernée.

3. Dispositif selon la revendication 1, caractérisé en ce que les moyens de transcription et d'enregistrement (2-7) comportent un système de reconnaissance vocale, les moyens de traitement (9) étant matérialisés par des moyens formant touches (8), par exemple ce système est programmé pour recevoir un message posologique transmis oralement, constitué de mots clefs tels que type de malade, quantité, forme galénique, et dictés suivant une série de séquences d'un protocole.

4. Dispositif selon l'une des revendications 1 à 3, caractérisé en ce que les moyens (1 à 7) de transcription et d'enregistrement et les moyens de traitement (9) comportent des moyens formant touches (8, 8a) correspondant chacune respectivement à un élément d'information composant la posologie du médicament, par exemple les touches (8) matérialisant les moyens (2) de transcription et d'enregistrement de la forme galénique du médicament sont au nombre de douze et comportent sur leur face externe un pictogramme représentant un comprimé, une gélule, une ampoule, un sachet, une dose, une cuillère à café, une cuillère à soupe, un ovule, un suppositoire, des gouttes, une pommade-crème, un pulvérisateur.

5. Dispositif selon l'une des revendications 1 à 4, caractérisé en ce qu'il comporte des moyens formant touche (8a) à plusieurs branches, notamment cruciformes, dont on peut presser chaque branche indépendamment des autres, par exemple, une première touche cruciforme (8a) a différentes branches correspondant respectivement à des heures approximatives de prise telles que 8 heures, 12 heures, 16 heures, 20 heures, et une seconde touche cruciforme (8a) a différentes branches permettant de préciser des moments de prise tels que avant le repas, pendant le repas, après le repas, au coucher, une troisième touche correspondant à la prescription matin/midi/soir.

6. Dispositif selon l'une des revendications 1 à 5, caractérisé en ce qu'il comporte un dispositif portatif susceptible d'être tenu dans une main et qu'il comprend en outre un moyen de visualisation (10) des éléments d'information et/ou de la posologie transcrits et enregistrés.

7. Dispositif selon l'une des revendications 1 à 6, caractérisé en ce qu'il est intégré dans un micro-ordinateur de gestion des approvisionnements et des stocks de médicaments.

8. Procédé, pour l'impression d'une étiquette adaptée à être fixée sur la boîte d'un médicament prescrit et comportant uniquement les éléments d'information nécessaires à la posologie prescrite de ce médicament, utilisant un dispositif selon l'une des revendications 1 à 7.

## Patentansprüche

1. An einen Apotheker gerichtete Vorrichtung für die Übertragung, die Aufzeichnung und die Wiedergabe der Posologie eines einer Privatperson verschriebenen Medikamentes, mit einem Elektronikmodul (1), das insbesondere Übertragungs- und Aufzeichnungsmittel (2) der galenischen Form des Medikamentes, Übertragungs- und Aufzeichnungsmittel (3) der Einnahmehäufigkeit des Medikamentes, Übertragungs- und Aufzeichnungsmittel (4) der einzunehmenden Menge des Medikamentes, Übertragungs- und Aufzeichnungsmittel (7) der Behandlungsdauer mit dem Medikament, Verarbeitungsmittel (9) der übertragenen und aufgezeichneten Informations- und/oder Posologieelemente, Übertragungs- und Aufzeichnungsmittel (5) des Einnahmezeitpunktes des Medikamentes, Übertragungs- und Aufzeichnungsmittel (6) der Art des behandelten Kranken und Druckmittel (11) steuert, dadurch gekennzeichnet, dass die Mittel (11) mit den Verarbeitungsmitteln (9) verbunden sind, um nur die Informationselemente zu drucken, die der Patient benötigt, um das Medikament nach der Verschreibung des Arztes einzunehmen, die auf ein Schild übertragen und auf diesem aufgezeichnet werden, das an der das Medikament enthaltenden Schachtel befestigt werden kann, wobei die Verarbeitungsmittel (6) so programmiert sind, dass sie standardmässig das «erwachsene » Informationselement wählen, um Überdosierungs-risiken des Medikamentes zu vermeiden.

2. Vorrichtung nach Anspruch 1, dadurch gekennzeichnet, dass das Elektronikmodul (1) einen Festspeicher und einen Mikroprozessor umfasst, die ein Übertragungsprotokoll erfordern, dessen Sequenzen dazu zwingen, nacheinander die folgenden Informationselemente einzugeben :
. Art des Kranken ;
. galenische Form des Medikamentes ;
. Menge ;
. Einnahmehäufigkeit ;
. Einnahmezeitpunkt ; und
. Behandlungsdauer ;
und das es nur diejenigen Informationselemente verzeichnet, die in einer vorgegebenen Form dargestellt sind, die mit der betroffenen Rubrik kompatibel ist.

3. Vorrichtung nach Anspruch 1, dadurch gekennzeichnet, dass die Übertragungs- und Aufzeichnungsmittel (2-7) ein Spracherkennungssystem umfassen, wobei die Verarbeitungsmittel (9) durch Tasten bildende Mittel (8) materialisiert sind ; dieses System ist beispielsweise programmiert, um eine mündlich übertragene posologische Meldung zu empfangen, die aus Schlüsselworten besteht, wie zum Beispiel Art des Kranken, Menge, galenische Form, die nach einer Sequenzreihe eines Protokolles diktiert werden.

4. Vorrichtung nach einem der Ansprüche 1 bis 3, dadurch gekennzeichnet, dass die Übertragungs- und Aufzeichnungsmittel (1 bis 7) und die Verarbeitungsmittel Tasten bildende Mittel (8, 8a) umfassen, die jeweils einem die Posologie des Medikamentes zusammensetzenden Informationselement entsprechen, beispielsweise die Tasten (8), die die Übertragungs- und Aufzeichnungsmittel (2) der galenischen Form des Medikamentes materialisieren, sind zwölf und umfassen an ihrer Aussenfläche ein Piktogramm, das eine Tablette, eine Kapsel, eine Ampulle, einen Beutel, eine Dosis, einen Teelöffel, einen Suppenlöffel, ein Ovul, ein Zäpfchen, Tropfen, eine Creme-Pommade, einen Zerstäuber, darstellt.

5. Vorrichtung nach einem der Ansprüche 1 bis 4, dadurch gekennzeichnet, dass sie Tasten bildende Mittel (8a) mit mehreren, insbesondere kreuzförmigen Zweigen umfasst, die man unabhängig voneinander drücken kann, wobei beispielsweise eine erste kreuzförmige Taste (8a) verschiedene Zweige hat, die jeweils den etwaigen Einnahmeuhrzeiten entsprechen, wie 8 Uhr, 12 Uhr, 16 Uhr, 20 Uhr, und eine zweite kreuzförmige Taste (8a) verschiedene Zweige hat, die Einnahmemomente angeben, wie vor der Mahlzeit, während der Mahlzeit, nach der Mahlzeit, beim Zubettgehen, während eine dritte Taste der Verschreibung morgens / mittags / abends entspricht.

6. Vorrichtung nach einem der Ansprüche 1 bis 5, dadurch gekennzeichnet, dass sie eine tragbare Vorrichtung umfasst, die in einer Hand gehalten werden kann, und dass sie weiters ein Anzeigemittel (10) der übertragenen und aufgezeichneten Informations- und/oder Posologieelemente aufweist.

7. Vorrichtung nach einem der Ansprüche 1 bis 6, dadurch gekennzeichnet, dass sie in einem Mikrocomputer integriert ist, der zur Verwaltung der Medikamentbeschaffungen und - bestände dient.

8. Druckverfahren eines Schilds zur Befestigung an der Schachtel eines verschriebenen Medikamentes, das nur die Informationselemente aufweist, die für die verschriebene Posologie dieses Medikamentes erforderlich sind, unter Verwendung einer Vorrichtung nach einem der Ansprüche 1 bis 7.

## Claims

1. A device intended to be used by a pharmacist, for transcribing, recording and reproducing the directions for use of a medicine prescribed for a member of the general public, comprising an electronic module (1) controlling notably means (2) of transcribing and recording the galenic form of the medicine, means (3) of transcribing and recording the frequency of taking the medicine, means (4) of transcribing and recording the quantity in which the medicine is to be taken, means (7) of transcribing and recording the duration of treatment by the medicine, means (9) of processing the items of information and/or the directions for use transcribed and recorded, means (5) for transcribing and recording the time at which the medicine needs to be taken, means (6) of transcribing and recording the type of patient being treated, and printing means (11), characterised in that the means (11) are connected to the processing means (9) for printing solely the items of information which the patient needs in order to take the medicine in accordance with the prescription from the doctor, transcribed and recorded on a label adapted to be fixed to the box containing this medicine, the processing means (6) being programmed so as to select by default the item of information "adult" in order to limit the risks of overdoses of the medicine.

2. A device according to Claim 1, characterised in that the electronic module (1) has a read only memory and a microprocessor which impose a transcription protocol whose sequences make it necessary to successively enter the following items of information:
• type of patient;
• galenic form of medicine;
• quantity;
• frequency of taking;
• time of taking; and
• duration of treatment;
and records only the items of information which are presented in a predetermined form compatible with the heading concerned.

3. A device according to Claim 1, characterised in that the transcription and recording means (2-7) have a voice recognition system, the processing means (9) being embodied by means forming keys (8), for example this system is programmed in order to receive a message on directions for use transmitted orally, consisting of key words such as type of patient, quantity or galenic form, and dictated in accordance with a series of protocol sequences.

4. A device according to one of Claims 1 to 3, characterised in that the transcription and recording means (1-7) and the processing means (9) include means forming keys (8, 8a) each corresponding respectively to an item of information making up the directions for use of the medicine, for example the keys (8) embodying the means (2) of transcribing and recording the galenic form of the medicine are twelve in number and have on their external face a pictogram representing a tablet, a capsule, an ampoule, a sachet, a dose, a teaspoon, a tablespoon, a pessary, a suppository, droplets, an ointment and a spray.

5. A device according to one of Claims 1 to 6, characterised in that it has means forming a key (8a) with several arms, notably cruciform, where each arm can be pressed independently of the others, for example a first cruciform key (8a) with different arms corresponding respectively to approximate times of taking such as 8 am, 12 noon, 4 pm or 8 pm, and a second cruciform key (8a) with different arms for specifying times of taking such as before meals, with meals, after meals or at bedtime, a third key corresponding to the instruction morning/midday/evening.

6. A device according to one of Claims 1 to 5, characterised in that it has a portable device able to be held in one hand and in that it also comprises a means (10) of displaying the items of information and/or the directions for use transcribed and recorded.

7. A device according to one of Claims 1 to 6, characterised in that it is integrated into a microcomputer for managing procurement and stocks of medicines.

8. A method for printing a label adapted to be fixed to the box of a prescribed medicine and including solely the items of information necessary to the directions for use prescribed for this medicine, using a device according to one of Claims 1 to 7.
